(19) Europäisches Patentamt
European Patent Office
Office européen des brevets

(11) Numéro de publication : **0 538 097 A1**

(12) # DEMANDE DE BREVET EUROPEEN

(21) Numéro de dépôt : **92402720.4**

(22) Date de dépôt : **06.10.92**

(51) Int. Cl.[5] : **C07D 277/30,** A01N 43/78, C07D 261/08, C07D 271/06, C07D 285/12, C07D 277/36, C07D 277/42, A01N 43/80, C07C 69/734, C07C 69/65, C07F 7/22, C07C 69/732, A01N 43/82

(30) Priorité : **11.10.91 FR 9112516**

(43) Date de publication de la demande : **21.04.93 Bulletin 93/16**

(84) Etats contractants désignés : **AT BE CH DE DK ES FR GB GR IT LI LU NL PT SE**

(71) Demandeur : **ROUSSEL-UCLAF
35, Boulevard des Invalides
F-75007 Paris (FR)**

(72) Inventeur : **Brayer, Jean-Louis
42, rue Jules Dubrulle
F-60440 Nanteuil le Haudoin (FR)**
Inventeur : **Demoute, Jean-Pierre
65, Avenue Foch
F-93360 Neuilly Plaisance (FR)**
Inventeur : **Laugraud, Sylvain
27, avenue des Cèdres
F-92410 Ville d'Avray (FR)**
Inventeur : **Reinier, Nicole
30, Chemin du Vallon de Toulouse
F-13009 Marseille (FR)**

(74) Mandataire : **Tonnellier, Marie-José et al
111, route de Noisy B.P. no 9
F-93230 Romainville (FR)**

(54) **Nouveaux dérivés de l'acide 1-naphtalène acétique, leur procédé de préparation et leur application comme pesticides.**

(57) L'invention a pour objet les composés de formule générale (I) :

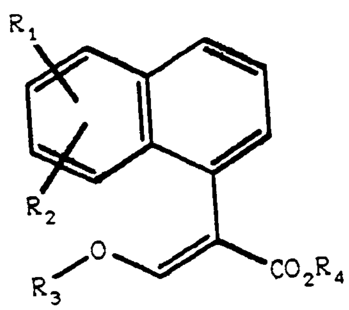

(I)

dans laquelle :
— $R_1$ = H, OH, Hal, $NO_2$, $NH_2$, CN, $CF_3$, $OCF_3$, $SCF_3$, $OR'_1$, $SR'_2$, $N(R'_3)_2$, $R'_1$, $R'_2$, $R'_3$, alkyle, alkényle, alkynyle ou aryle éventuellement substitué,
— $R_2$ = hétérocycle renfermant au moins 2 hétéroatomes éventuellement substitués,
— $R_3$ et $R_4$, identiques ou différents = alkyle éventuellement substitué.
Les composés de formule (I) présentent d'intéressantes propriétés pesticides.

EP 0 538 097 A1

La présente invention concerne de nouveaux dérivés de l'acide 1-naphtalène acétique, leur procédé de préparation et leur application comme pesticides.

L'invention a pour objet les composés de formule générale (I) :

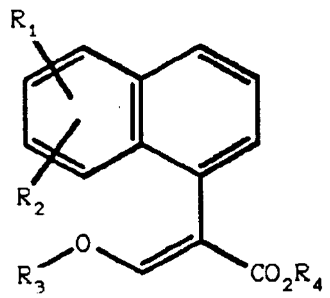

(I)

dans laquelle :

- $R_1$ représente un atome d'hydrogène, un radical hydroxyle, un atome d'halogène, un radical $NO_2$, $NH_2$, C≡N, $CF_3$, $OCF_3$ ou $SCF_3$, un radical $OR'_1$, $SR'_2$ ou $N(R'_3)_2$, $R'_1$, $R'_2$ et $R'_3$ représentant un radical alkyle, alkényle ou alkynyle renfermant jusqu'à 8 atomes de carbone ou un radical aryle renfermant jusqu'à 14 atomes de carbone, éventuellement substitué par un ou plusieurs substituants choisis parmi les atomes d'halogène et les radicaux $NO_2$, $NH_2$, C≡N, $CF_3$, $OCF_3$ et $SCF_3$,
- $R_2$ représente un radical hétérocyclique à 5 chaînons renfermant au moins 2 hétéroatomes et relié au noyau naphtyl par une liaison carbone-carbone, éventuellement substitué par un ou plusieurs atomes groupes Z, Z représentant un atome d'halogène, un radical alkyle, alkényle ou alkynyle renfermant jusqu'à 8 atomes de carbone linéaire ou ramifié, un radical hydroxyle, $NO_2$, $NH_2$, C≡N, $CF_3$, $OCF_3$ ou $SCF_3$, $OR''_1$, $SR''_2$, $SOR''_2$, $SO_2R''_2$, $NR''_3$, $R''_1$, $R''_2$ et $R''_3$ représentant un radical alkyle, alkényle ou alkynyle renfermant jusqu'à 8 atomes de carbone ou Z représente un radical hétérocyclique à 5 ou 6 chaînons ou un radical aryle renfermant jusqu'à 14 atomes de carbone éventuellement substitué par un ou plusieurs substituants choisis parmi les atomes d'halogène et les radicaux $NO_2$, $NH_2$, C≡N, $CF_3$, $OCF_3$, $SCF_3$, un radical méthylènedioxy ou un radical $OR''_1$, $SR''_2$, $SOR''_2$, $SO_2R''_2$ ou $NR''_3$, $R''_1$, $R''_2$ et $R''_3$ conservant leur signification précédente,
- $R_3$ et $R_4$, identiques ou différents, représentent un radical alkyle renfermant jusqu'à 8 atomes de carbone linéaire, ramifié ou cyclique éventuellement substitué par un ou plusieurs atomes d'halogène.

Dans la définition des divers substituants,

- alkyle représente de préférence un radical méthyle, éthyle, propyle, isopropyle, butyle, isobutyle, sec-butyle ou tert-butyle,
- alkényle représente de préférence un radical vinyle, allyle ou 1,1-diméthylallyle,
- alkynyle représente de préférence un radical éthynyle ou propynyle, aryle représente de préférence le radical phényle,
- hétérocyclique représente de préférence un radical thiazolyle, oxazolyle, isothiazolyle, isoxazolyle, thiadiazolyle ou oxadiazolyle (1,2,4 ou 1,3,4), triazolyle, imidazolyle ou pyrazolyle,
- halogène représente de préférence un atome de fluor ou de chlore.

Parmi les composés préférés de l'invention, on peut citer

- les composés dans lesquels $R_3$ représente un radical méthyle,
- les composés dans lesquels $R_4$ représente un radical méthyle,
- les composés dans lesquels $R_1$ représente un atome d'hydrogène,
- les composés dans lesquels $R_2$ représente un radical thiazolyle, isoxazolyle ou oxadiazyle éventuellement substitué par un ou plusieurs des substituants indiqués précédemment.

Parmi les composés préférés de l'invention, on peut citer plus particulièrement les composés dans lesquels $R_2$ représente un radical thiazolyle substitué par un radical phényle substitué par un ou plusieurs atomes d'halogène ou par un ou plusieurs radicaux alkyle ou alkoxy éventuellement substitués par un ou plusieurs atomes d'halogène, et notamment ceux dans lesquels $R_2$ représente un radical thiazolyle substitué par un radical phényle substitué par un ou plusieurs atomes de fluor ou de chlore, par un ou plusieurs radicaux $CF_3$ ou $OCF_3$.

Parmi les composés préférés de l'invention, on peut également citer plus particulièrement les composés de formule (I) dans lesquels $R_2$ représente un radical thiazolyle substitué par un ou plusieurs radicaux alkyles éventuellement substitués par un ou plusieurs atomes d'halogène notamment ceux dans lesquels $R_2$ repré-

sente un radical thiazolyle substitué par un radical $CF_3$, les composés de formule (I) dans lesquels $R_2$ représente un radical bithiazolyle éventuellement substitué par un ou plusieurs radicaux alkyle, les composés de formule (I) dans lesquels $R_2$ représente un radical isoxazolyle substitué par un ou plusieurs radicaux alkyles éventuellement substitués par un ou plusieurs atomes d'halogène, ainsi que les composés de formule (I) dans lesquels $R_2$ représente un radical oxadiazyle substitué par un ou plusieurs radicaux alkyles éventuellement substitués par un ou plusieurs atomes d'halogène.

Parmi les composés préférés de l'invention, on peut citer tout particulièrement les composés dont la préparation est donnée ci-après dans la partie expérimentale et tout spécialement les composés de formule (I) dont les noms suivent :

- (E) 7-[2-(3,4-dichlorophényl) 4-thiazolyl] α-(méthoxyméthylène) 1-naphtalène acétate de méthyle,
- (E) α-(méthoxyméthylène) 7-[2-(trifluorométhyl) 4-thiazolyl] 1-naphtalène acétate de méthyle,
- (E) 7-[2-(4-fluorophényl) 4-thiazolyl] α-(méthoxyméthylène) 1-naphtalène acétate de méthyle,
- (E) 7-[2-[4-(trifluorométhoxy) phényl] 4-thiazolyl] α-(méthoxyméthylène) 1-naphtalène acétate de méthyle,
- (E) α-(méthoxyméthylène) 7-[3-(trifluorométhyl) 5-isoxazolyl) 1-naphtalène acétate de méthyle,
- (E) α-(méthoxyméthylène) 7-[2'-(1-méthyléthyl) 2,4'-bithiazole) 4-yl] 1-naphtalène acétate de méthyle,
- (E) α-(méthoxyméthylène) 7-[5-(trifluorométhyl) 1,2,4-oxadiazol-3-yl] 1-naphtalène acétate de méthyle.

Les composés de formule (I) présentent d'intéressantes propriétés qui permettent leur utilisation dans la lutte contre les parasites. Il peut s'agir par exemple de la lutte contre les parasites des végétaux, qu'il s'agisse des parasites du sol ou des parties aériennes, les parasites des locaux et les parasites des animaux à sang chaud.

C'est ainsi que l'on peut utiliser les produits de l'invention pour lutter contre les insectes, les nématodes et les acariens parasites des végétaux et des animaux.

L'invention a notamment pour objet l'application des composés de formule (I) à la lutte contre les parasites des végétaux, les parasites des locaux et les parasites des animaux à sang chaud.

Les produits de formule (I) peuvent également être utilisés pour lutter contre les insectes dans les locaux, pour lutter notamment contre les mouches, les moustiques et les blattes.

Les produits de formule (I) peuvent aussi être utilisés pour lutter contre les insectes et autres parasites du sol, par exemple les coléoptères, comme Diabrotica, les taupins et les vers blancs, les myriapodes comme les scutigérelles et les blaniules, et les diptères comme les cécydomies et les lépidoptères comme les noctuelles terricoles.

Ils sont utilisés à des doses comprises entre 5 g et 300 g de matière active à l'hectare.

L'ensemble de ces propriétés fait des produits de formule (I) des produits qui correspondent parfaitement aux exigences de l'industrie agrochimique moderne : ils permettent de protéger les récoltes tout en préservant l'environnement.

Les produits de formule (I) peuvent aussi être utilisés pour lutter contre les acariens et les nématodes parasites des végétaux.

Les composés de formule (I) peuvent encore être utilisés pour lutter contre les acariens parasites des animaux, pour lutter par exemple contre les tiques et notamment les tiques du genre de Boophilus, ceux du genre Hyalomnia, ceux du genre Amblyomnia et ceux du genre Rhipicephalus ou pour lutter contre toutes sortes de gales et notamment la gale sarcoptique, la gale psoroptique et la gale chorioptique.

L'invention a donc également pour objet les compositions destinées à la lutte contre les parasites des animaux à sang chaud, les parasites des locaux et des végétaux, caractérisées en ce qu'elles renferment au moins l'un des produits de formule (I) définis ci-dessus et notamment l'un des produits des exemples 1, 2, 3, 5, 8, 20 et 22.

L'invention a notamment pour objet les compositions insecticides renfermant comme principe actif au moins l'un des produits définis ci-dessus.

Ces compositions sont préparées selon les procédés usuels de l'industrie agrochimique ou de l'industrie vétérinaire ou de l'industrie des produits destinés à la nutrition animale.

Dans ces compositions destinées à l'usage agricole et à l'usage dans les locaux, la ou les matières actives peuvent être additionnées éventuellement d'un ou plusieurs autres agents pesticides. Ces compositions peuvent se présenter sous forme de poudres, granulés, suspensions, émulsions, solutions, solutions pour aérosols, bandes combustibles, appâts ou autres préparations employées classiquement pour l'utilisation de ce genre de composés.

Outre le principe actif, ces compositions contiennent, en général, un véhicule et/ou un agent tensio-actif, non ionique, assurant, en outre, une dispersion uniforme des substances constitutives du mélange. Le véhicule utilisé peut être un liquide, tel que l'eau, l'alcool, les hydrocarbures ou autres solvants organiques, une huile minérale, animale ou végétale, une poudre telle que le talc, les argiles, les silicates, le kieselguhr ou un solide

combustible.

Les compositions insecticides selon l'invention contiennent de préférence de 0,005 % à 10 % en poids de matière active.

Selon un mode opératoire avantageux, pour un usage dans les locaux, les compositions selon l'invention sont utilisées sous forme de compositions fumigantes.

Les compositions selon l'invention peuvent alors être avantageusement constituées, pour la partie non active, d'un serpentin insecticide (ou coil) combustible, ou encore d'un substrat fibreux incombustible. Dans ce dernier cas, le fumigant obtenu après incorporation de la matière active est placé sur un appareil chauffant tel qu'un émanateur électrique.

Dans le cas où l'on utilise un serpentin insecticide, le support inerte peut être, par exemple, composé de marc de pyrèthre, poudre de Tabu (ou poudre de feuilles Machilus Thumbergii), poudre de tige de pyrèthre, poudre de feuille de cèdre, poudre de bois (telle que de la sciure de pin) amidon et poudre de coque de noix de coco.

La dose de matière active peut alors être, par exemple, de 0,03 à 1 % en poids.

Dans le cas où l'on utilise un support fibreux incombustible, la dose de matière active peut alors être, par exemple, de 0,03 à 95 % en poids.

Les compositions selon l'invention pour un usage dans les locaux peuvent aussi être obtenues en préparant une huile pulvérisable à base de principe actif, cette huile imbibant la mèche d'une lampe et étant alors soumise à la combustion.

La concentration du principe actif incorporé à l'huile est, de préférence, de 0,03 à 95 % en poids.

Les compositions insecticides selon l'invention, comme les compositions acaricides et nématicides, peuvent être additionnées éventuellement d'un ou plusieurs autres agents pesticides. Les compositions acaricides et nématicides peuvent se présenter notamment sous forme de poudre, granulés, suspensions, émulsions, solutions.

Pour l'usage acaricide, on utilise de préférence des poudres mouillables, pour pulvérisation foliaire, contenant de 1 à 80 % en poids de matière active ou des liquides pour pulvérisation foliaire contenant de 1 à 500 g/l de principe actif. On peut également employer des poudres pour poudrages foliaires contenant de 0,05 à 3 % de matière active.

Pour l'usage nématicide, on utilise de préférence des liquides pour traitement des sols contenant de 300 à 500 g/l de principe actif.

Les composés acaricides et nématicides selon l'invention sont utilisés, de préférence, à des doses comprises entre 1 et 100 g de matière active à l'hectare.

Pour exalter l'activité biologique des produits de l'invention on peut les additionner à des synergistes classiques utilisés en pareil cas tel que le 1-(2,5,8-trioxadodécyl) 2-propyl 4,5-méthylènedioxy benzène (ou butoxyde de pipéronyle) ou la N-(2-éthyl heptyl) bicyclo[2.2.1]hept-5-ène-2,3-dicarboximide, ou le pipéronyl-bis-2-(2'-n-butoxy éthoxy) éthylacétal (ou tropital).

Les composés de formule (I) présentent une excellente tolérance générale, et l'invention a donc également pour objet les produits de formule (I), pour lutter notamment contre les affections créées par les tiques et les gales chez l'homme et l'animal.

Les produits de l'invention sont notamment utilisés pour lutter contre les poux à titre préventif ou curatif et pour lutter contre la gale.

Les produits de l'invention peuvent être administrés par voie externe, par vaporisation, par shampooing, par bain ou badigeonnage.

Les produits de l'invention à usage vétérinaire peuvent être également administrés par badigeonnage de l'épine dorsale selon la méthode dite méthode "pour-on".

On peut indiquer également que les produits de l'invention peuvent être utilisés comme biocides ou comme régulateurs de croissance.

L'invention a également pour objet les associations douées d'activité insecticide, acaricide ou nématicide, caractérisées en ce qu'elles contiennent comme matière active, d'une part un au moins des composés de formule générale (I), et d'autre part, un au moins des esters pyréthrinoïdes choisis dans le groupe constitué par les esters d'alléthrolone, d'alcool 3,4,5,6-tétrahydrophtalimidométhylique, d'alcool 5-benzyl 3-furyl méthylique, d'alcools 3-phénoxy benzylique et d'alcools alpha-cyano 3-phénoxy benzyliques des acides chrysanthémiques, par les esters d'alcools 5-benzyl 3-furyl méthylique des acides 2,2-diméthyl 3-(2-oxo 3-tétrahydrothiophénylidène méthyl) cyclopropanecarboxyliques, par les esters d'alcool 3-phénoxy benzylique et d'alcools alpha-cyano 3-phénoxy benzyliques des acides 2,2-diméthyl 3-(2,2-dichlorovinyl) cyclopropanecarboxyliques, par les esters d'alcools alpha-cyano 3-phénoxy benzyliques d'acides 2,2-diméthyl 3(2,2-dibromovinyl) cyclopropanecarboxyliques, par les esters d'alcools 3-phénoxy benzylique des acides 2-parachlorophényl 2-isopropyl acétiques, par les esters d'alléthrolones, d'alcool 3,4,5,6-tétrahydrophtalimidométhylique, d'alcool 5-benzyl 3-furyl méthylique, d'alcool 3-phénoxy benzylique et d'alcools alpha-cyano 3-phénoxy benzyliques des acides 2,2-diméthyl 3-(1,2,2,2-tétrahaloéthyl) cyclopropanecarboxyliques, dans lesquels "halo" représente un atome de fluor, de chlore ou de brome, étant entendu que les composés de formule (I) peuvent exister sous toutes leurs formes stéréoisomères possibles de même que les copules acides et alcools des esters pyréthrinoïdes ci-dessus.

L'invention a également pour objet un procédé de préparation des composés de formule (I) caractérisé en ce que l'on soumet un composé de formule (II) :

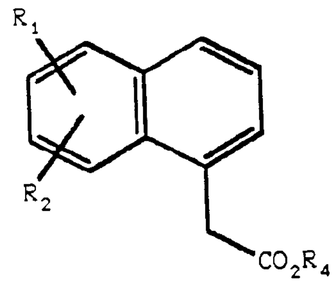

(II)

dans laquelle $R_1$, $R_2$ et $R_4$ conservent la même signification que précédemment, à l'action d'un agent de formylation puis à l'action d'un agent d'alkylation susceptible d'introduire le radical $R_3$, $R_3$ étant défini comme précédemment pour obtenir le composé de formule (I) correspondant.

Dans un mode de réalisation préféré, l'agent de formylation est un formiate d'alkyle en présence d'une base forte ou encore un acétal du diméthylformamide ou les bis dialkylamino terbutoxy méthane en chauffant à température du reflux puis en faisant réagir sur l'énamine intermédiaire avec un acide tel que l'acide chlorhydrique au sein d'un solvant organique en milieu aqueux tel que le tétrahydrofuranne aqueux.

La base forte utilisée avec le formiate d'alkyle est l'hydrure de sodium ou encore un alcoolate alcalin ou alcalinoterreux, un amidure alcalin, une amine secondaire ou tertiaire en présence de bromure de lithium,
- l'agent d'alkylation susceptible d'introduire le radical $R_3$ est un dérivé $HalR_3$ dans lequel Hal représente par exemple un atome d'iode ou de chlore ou encore un sulfate $SO_4(R_3)_2$.

L'invention a également pour objet un procédé de préparation des composés de formule (I) caractérisé en ce que l'on soumet un produit de formule (II') :

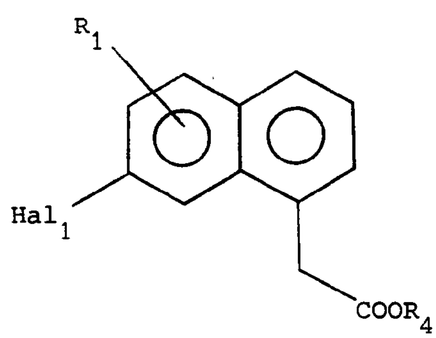

(II')

dans laquelle $R_1$ a la signification indiquée précédemment à l'exception d'un atome d'halogène, $R_4$ a la signification déjà indiquée et $Hal_1$ représente un atome d'halogène à l'action d'un agent de formylation puis à l'action d'un agent d'alkylation susceptible d'introduire le radical $R_3$, $R_3$ étant défini comme précédemment pour obtenir le composé de formule (III) correspondant :

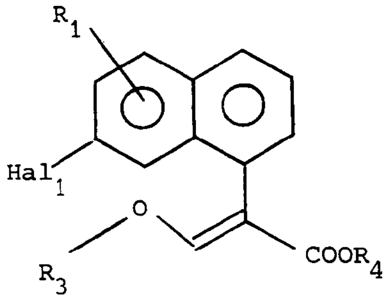

(III)

dans laquelle $R_1$, $R_3$, $R_4$ et $Hal_1$ ont la signification déjà indiquée, que l'on soumet à l'action d'un réactif stannylé en présence d'un catalyseur au palladium et d'un dérivé de triphénylphosphine pour obtenir un produit de formule (IV) :

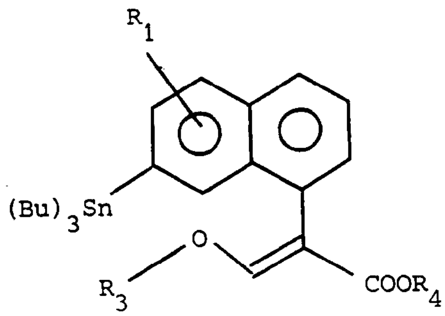

(IV)

dans laquelle $R_1$, $R_3$ et $R_4$ ont la signification déjà indiquée que l'on soumet en présence d'un catalyseur au palladium et d'un dérivé de triphénylphosphine à l'action d'un produit de formule (V) :

$R_2$-X (V)

dans laquelle X représente un atome d'halogène et $R_2$ a la signification indiquée précédemment, pour obtenir le composé de formule (I) correspondant.

Dans un mode de réalisation préféré, l'atome d'halogène que représente Hal peut être un atome d'iode mais de préférence de brome.

- L'agent de formylation et l'agent d'alkylation susceptible d'introduire le radical $R_3$ peuvent être choisis parmi ceux indiqués pour la réaction sur les produits de formule (II).
- Le dérivé stannylé utilisé est par exemple l'hexabutyldistannane au sein d'un solvant organique anhydre tel que le toluène en présence de tétrakis(triphénylphosphine) palladium.
- L'atome d'halogène du produit de formule $R_2X$ est de préférence un atome de brome. La réaction du produit de formule $R_2X$ sur le produit de formule (IV) est effectué au sein d'un solvant organique tel que le dioxanne en présence de chlorure de bis(triphénylphosphine) palladium.

L'invention a également pour objet un procédé de préparation des produits de formule (I) dans laquelle $R_1$, $R_3$ et $R_4$ ont la signification indiquée ci-dessus et $R_2$ représente un radical isoxazolyle éventuellement substitué par un groupe Z dans lequel Z a la signification indiquée précédemment, caractérisé en ce que l'on soumet un produit de formule (VI) :

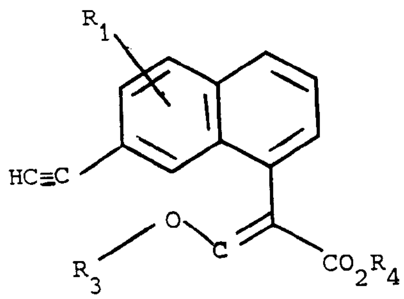

(VI)

en milieu basique, à l'action d'une oxime de formule

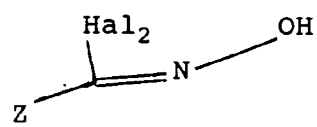

dans laquelle $Hal_2$ représente un atome d'halogène et Z a la signification indiquée ci-dessus.

Dans un mode de réalisation préféré, l'atome d'halogène que représente $Hal_2$ est un atome de chlore ou de brome, Z représente un radical alkyle linéaire ou ramifié renfermant jusqu'à 6 atomes de carbone éventuellement substitué par un ou plusieurs atomes d'halogène.

L'agent basique utilisé est par exemple la triéthylamine au sein d'un solvant organique tel que le tétrahydrofuranne.

Les composés de formule (II) sont des produits chimiques nouveaux et sont en eux-mêmes un objet de la présente invention, leur préparation peut être schématisée comme suit :

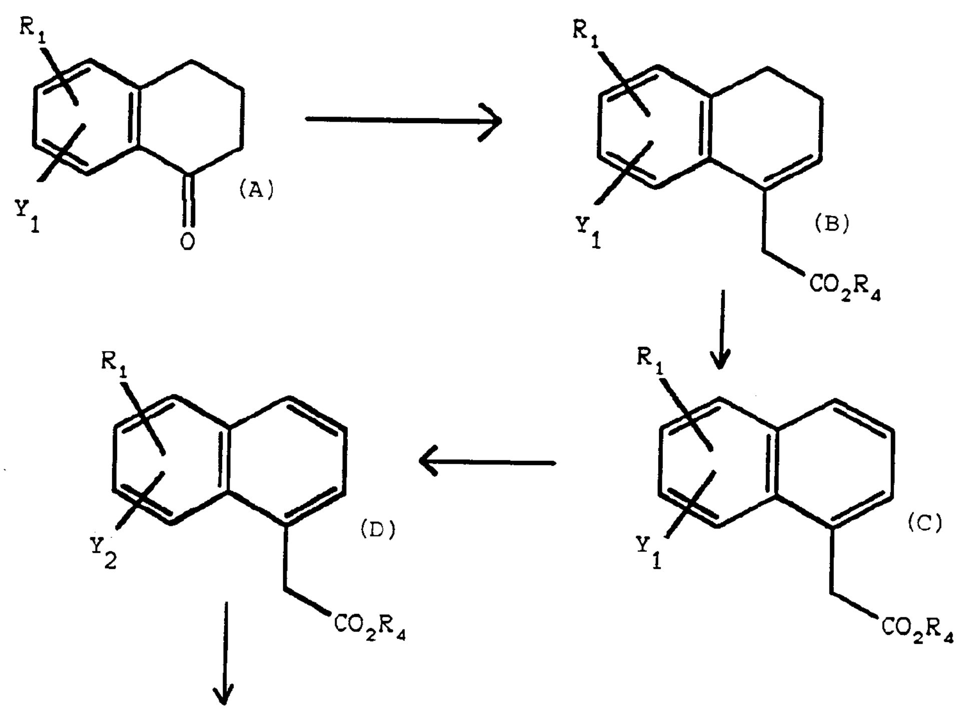

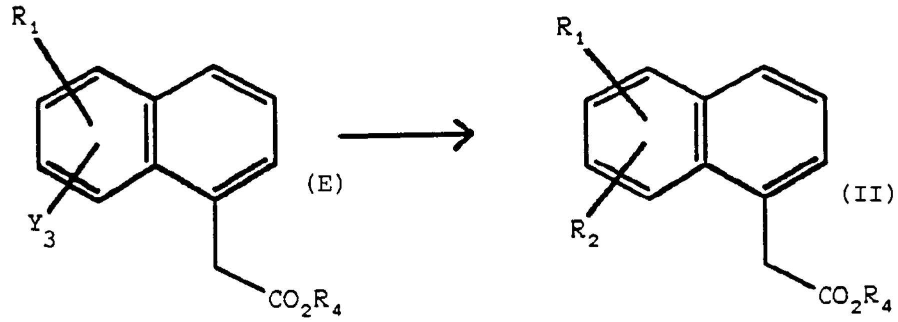

(E) (II)

$Y_1$ représente I, Br ou $NO_2$
$Y_2$ représente

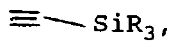

$COCH_3$, C≡N
$Y_3$ représente C≡C, $COCH_2Br$, $CONH_2$, $CSNH_2$, CO-NH-$NH_2$, CSNH-$NH_2$, $CNH_2$-NHOH, COOH.

Les produits de formule (II') peuvent être obtenus comme indiqué aux stades A, B et C de la synthèse décrite ci-dessus.

Les composés de formule (VI) sont des produits décrits ou préparés comme indiqué dans la demande française 92 04565 déposé par la demanderesse le 14 avril 1992.

Des exemples détaillés de préparation des composés de formules (II), (II') et (VI) sont donnés ci-après dans la partie expérimentale.

L'invention a plus particulièrement pour objet à titre de produits chimiques nouveaux, les composés de formules (II), (II'), (III) et (IV) dont la préparation est donnée ci-après dans la partie expérimentale.

**EXEMPLE 1 : (E) 7-[2-(3,4-dichlorophényl) 4-thiazolyl] α-(méthoxyméthylène) 1-naphtalène acétate de méthyle**

On ajoute, sous atmosphère d'azote, une solution renfermant 0,36 g du produit de la préparation 1, 0,41 $cm^3$ de formiate de méthyle et 6 $cm^3$ de diméthylformamide anhydre dans une suspension renfermant 0,1 g d'hydrure de sodium à 50 % dans l'huile et 6 $cm^3$ de diméthylformamide anhydre. On maintient le mélange réactionnel sous agitation pendant 1 heure à 20°C et ajoute 0,1 $cm^3$ de formiate de méthyle. On agite pendant 4 heures, verse le mélange réactionnel dans l'eau, acidifie à pH ≃ 2 avec de l'acide chlorhydrique 2N et extrait à l'éther, lave à l'eau avec une solution saturée en chlorure de sodium, sèche sur sulfate de magnésium, et évapore sous pression réduite. On obtient un produit brut que l'on dissout dans 8 $cm^3$ d'acétone anhydre. On ajoute 0,12 $cm^3$ de diméthylsulfate et 0,35 g de carbonate de potassium. On maintient le mélange réactionnel sous agitation pendant 1 h 30 à 20°C, le verse dans l'eau et extrait à l'éther. On lave à l'eau, sature de chlorure de sodium, sèche sur sulfate de magnésium et évapore sous pression réduite. On obtient un produit que l'on chromatographie sur silice en éluant avec le mélange hexane-acétate d'éthyle (8-2). On obtient après recristallisation dans l'hexane 0,22 g de produit recherché. F = 173°C.

Spectre RMN ($CDCl_3$ 250 MHz)

| | |
|---|---|
| Les 2 méthoxy | 3,71 (s) |
| | 3,83 (s) |
| -C=C-de Delta E | 7,59 (s) |
| et $H_5$ thiazole | 7,85 (s) |
| $H_{3'}$ et $H_1$ | 8,18 (d) |
| | 8,32 (sl) |

**PREPARATION 1 : 7-[2-(3,4-dichlorophényl) 4-thiazolyl] 1-naphtalène acétate de méthyle**

Stade A : 7-bromo 3,4-dihydro 1-naphtalène acétate de méthyle

On chauffe à 60°C une suspension renfermant 5,8 g de poudre de zinc activée (c'est-à-dire obtenue après lavages à l'acide chlorhydrique à 20 % (en volume) puis à 5 % puis à l'eau, puis à l'éthanol et enfin à l'éther éthylique et séchage), 70 $cm^3$ de tétrahydrofuranne anhydre, 1 cristal d'iode et 0,1 $cm^3$ de bromoacétate de

méthyle. On ajoute sous atmosphère d'azote, une solution renfermant 10 g de 7-bromotétralone (préparé selon J. Org. Chem., 27, 76 (1962)) 5 $cm^3$ de bromoacétate de méthyle et 60 $cm^3$ de tétrahydrofuranne anhydre. On agite ensuite le milieu réactionnel à 20°C pendant 2 heures, verse dans l'eau, neutralise par adjonction de chlorure d'ammonium et extrait à l'éther. On filtre la solution aqueuse et réextrait à l'éther. On rassemble les phases organiques, les lave avec une solution aqueuse saturée en chlorure de sodium, sèche et évapore sous pression réduite. On obtient 13,6 g d'un produit que l'on dissout dans 90 $cm^3$ d'acide trifluoroacétique. On chauffe à 50°C pendant 30 minutes et verse dans une solution refermant 1 l d'eau et 500 $cm^3$ d'éther. On ajoute du carbonate de potassium jusqu'à un pH voisin de 10. On décante, lave avec une solution aqueuse saturée en chlorure de sodium, sèche et évapore sous pression réduite. On obtient 11,8 g de produit que l'on chromatographie sur silice en éluant avec un mélange hexane-éther isopropylique (9-1) puis avec un mélange hexane-éther isopropylique (7-3). On obtient 9,9 g du produit recherché.

CCM (silice) éluant : hexane-éther isopropylique (8-2) rf = 0,38.

Spectre RMN (250 MHz)

| | |
|---|---|
| $CH_2$ en 3 | ≃ 2,32 (m) |
| $CH_2$ en 4 | 2,73 (t) |
| $=C-CH_2-C=O$ | 3,41 (s) |
| $CO_2CH_3$ | 3,71 (s) |
| $H_2$ | 6,04 (t) |
| $H_5$ | 7,00 (d) |
| $H_8 + H_6$ | ≃ 7,25 (m) |

Stade B : 7-bromo 1-naphtalène acétate de méthyle

On chauffe à 90°C pendant 3 h 30 minutes une solution renfermant 9,85 g de produit préparé au stade A avec 10,3 g de dichlorodicyanobenzoquinone et 300 $cm^3$ de toluène. On maintient le mélange réactionnel pendant 12 heures à 20°C. On filtre, et évapore sous pression réduite pour obtenir 12,7 g de produit que l'on chromatographie sur silice en éluant avec le mélange hexane-chlorure de méthylène (7-3). On obtient ainsi 5,1 g de produit recherché. F = 61°C.

Spectre RMN (250 MHz)

| | |
|---|---|
| $COOCH_3$ | 3,66 (s) |
| $=C_6H_3-CH_2-CO$ | 3,98 (s) |
| $H_8$ | 8,1 (sl) |
| | 7,42 (m) 2H |
| H aromatiques | 7,52 (dd) 1H |
| | 7,68 (m) 2H |

Stade C : 7-[2-(triméthylsilyl) éthynyl] 1-naphtalène acétate de méthyle

On chauffe à reflux sous atmosphère d'azote pendant 3 h 30, une solution renfermant 40 $cm^3$ de triéthylamine, 20 $cm^3$ d'acétonitrile anhydre, 4,5 g de produit préparé au stade précédent, 2,35 $cm^3$ de triméthylsilylacétylène, 0,68 g de palladium à 10 % sur charbon humide, 0,68 g de triphénylphosphine et 0,12 g d'iodure de cuivre. On évapore sous pression réduite, on reprend au chlorure de méthylène, lave avec une solution aqueuse saturée en chlorure de sodium, sèche et évapore sous pression réduite. On chromatographie le résidu obtenu sur silice en éluant avec le mélange hexane-éther isopropylique (8-2). On obtient ainsi 4,33 g de produit recherché. F = 78°C.

Spectre RMN (250 MHz)

| | |
|---|---|
| $Si(CH_3)_3$ | 0,29 (s) |
| $COOCH_3$ | 3,70 (s) |
| $=C_6H_3-CH_2-COO$ | 4,06 (s) |
| Aromatiques | 7,38 à 7,80 (5H) |
| | 8,11 (sl) $H_8$ |

Stade D : 7-(acétyl) 1-naphtalène acétate de méthyle

On ajoute 0,55 $cm^3$ d'acide sulfurique concentré puis 0,45 g de sulfate mercurique dans une solution renfermant 1,52 g de produit préparé au stade précédent et 25 $cm^3$ d'un mélange acétone-eau (9-1). On agite la solution résultante pendant 2 heures à 20°C. On verse le milieu réactionnel dans l'eau, extrait au chlorure de méthylène, lave à l'eau puis avec une solution aqueuse saturée en chlorure de sodium. On sèche et évapore

sous pression réduite. On chromatographie le produit obtenu sur silice en éluant avec le mélange hexaneacétate d'éthyle (75-25). On obtient ainsi 0,91 g de produit recherché. F = 110°C.

Spectre RMN (250 MHz) $CDCl_3$

| | |
|---|---|
| $CH_3CO$ | 2,75 (s) |
| $CH_3OCO$ | 3,71 (s) |
| $C_6H_3$-$CH_2$-CO | 4,15 (s) |
| $H_3$ | 7,49 (t,J = 7 Hz) |
| $H_2$ + $H_4$ | 7,56 (d,J = 7 Hz) 7,83 (d,J = 8 Hz) |
| $H_5$ | 7,92 (d,J = 8,5 Hz) |
| $H_6$ | 8,05 (dd; J = 1,5 Hz ; J = 8,5 Hz) |
| $H_8$ | 8,69 (sl) |

<u>Stade E</u> : 7-[2-(3,4-dichlorophényl) 4-thiazolyl] 1-naphtalène acétate de méthyle

<u>Stade E1</u> :

On ajoute 0,66 g de perbromure de bromure de diméthylamino 4-pyridinium dans une solution renfermant 0,4 g de produit préparé au stade précédent et 7 cm³ d'acide acétique glacial. On agite la suspension ainsi obtenue pendant 4 heures à 20°C et dilue avec du chlorure de méthylène. On lave à l'eau, avec une solution aqueuse saturée en carbonate acide de sodium, puis avec une solution aqueuse saturée en chlorure de sodium. On sèche sur sulfate de magnésium et évapore sous pression réduite. On obtient 0,55 g de produit.

<u>Stade E2</u> :

On chauffe au reflux pendant 40 minutes une solution renfermant 0,55 g du produit préparé au stade E1, 0,51 g de dichloro 3,4-thiobenzamide et 15 cm³ de méthanol. On maintient le mélange réactionnel sous agitation pendant 12 heures à 20°C. On filtre la suspension obtenue et rince le produit obtenu à l'hexane. On obtient ainsi 0,4 g de produit recherché. rf = 0,41 éluant hexane-acétate d'éthyle (7-3).

Spectre RMN $CDCl_3$ (250 MHz)

| | |
|---|---|
| $OCH_3$ | 3,74 (s) |
| =C-$CH_2$-C=O | 4,18 (s) |
| $H_3$; $H_4$ et $H_2$ | 7,45 (m) (2H) |
| | 7,82 (m) 1H |
| $H_5''$ | 7,55 (d,J = 8,5 Hz) |
| $H_5'$ thiazole | 7,66 (s) |
| $H_6''$ | 7,88 (d,d J=2-8,5 Hz) |
| $H_5$ | 7,93 (d,J = 8,5 Hz) |
| $H_6$ | 8,07 (dd,J = 1,5 - 8,5) |
| $H_2''$ | 8,22 (d,J = 2Hz) |
| $H_8$ | 8,67 (l) |

**<u>EXEMPLE 2</u> : (E) α-(méthoxyméthylène) 7-[2-(trifluorométhyl) 4-thiazolyl] 1-naphtalène acétate de méthyle**

On introduit une solution renfermant 0,29 g de produit de la préparation 2, 0,5 cm³ de formiate de méthyle et 8 cm³ de diméthylformamide anhydre, dans une suspension renfermant 0,1 g d'hydrure de sodium à 50 % dans l'huile et 5 cm³ de diméthylformamide anhydre. On maintient le mélange réactionnel sous agitation pendant 1 h 30, ajoute 0,5 cm³ de formiate de méthyle et maintient à nouveau l'agitation pendant 12 heures. On verse le milieu réactionnel dans l'eau, acidifie à pH ≃ 2 et extrait à l'éther. On rassemble les phases organiques, les lave avec une solution aqueuse saturée de chlorure de sodium, sèche sur sulfate de magnésium et évapore sous pression réduite. On dissout le produit ainsi obtenu dans 7 cm³ d'acétone anhydre. On ajoute 0,1 cm³ de diméthylsulfate et 0,35 g de carbonate de potassium. On agite la suspension résultante pendant 12 heures à 20°C, la verse dans l'eau et extrait à l'éther. On lave la phase organique avec une solution aqueuse saturée en chlorure de sodium, sèche sur sulfate de magnésium et évapore sous pression réduite. On chromatographie le produit obtenu sur silice en éluant avec le mélange flugène 113-acétate d'éthyle (98-2). On obtient ainsi 0,16 g de produit recherché. F = 162°C.

Spectre RMN ($CDCl_3$ 250 MHz)

Les 2 méthoxy 3,70 (s)

| | |
|---|---|
| | 3,83 (s) |
| $H_5$ thiazole | 7,76 (s) |
| CH= | 7,84 (s) |
| | 7,38 (dl) 1H |
| | 7,53 (t) 1H |
| H aromatiques | 7,8 à 8,0 (m) 3H |
| | 8,29 (sl) 1H |

**PREPARATION 2 : 7-[2-(trifluorométhyl) 4-thiazolyl] 1-naphtalène acétate de méthyle**

On chauffe au reflux pendant 16 heures une solution renfermant 3,1 g de produit préparé au stade E1 de la préparation 1, 3 g de trifluorothioacétamide et 60 $cm^3$ de méthanol. On verse le mélange réactionnel dans une solution aqueuse saturée en carbonate acide de sodium et extrait au chlorure de méthylène. On sèche la phase organique et évapore sous pression réduite. On chromatographie le produit obtenu sur silice en éluant avec le mélange hexane-chlorure de méthylène (1-1). On obtient 1,2 g du produit recherché. F = 140°C.

Spectre RMN ($CDCl_3$) 250 MHz

| | |
|---|---|
| $OCH_3$ | 3,72 (s) |
| $=C-CH_2-C=O$ | 4,16 (s) |
| $H_5'$ thiazole | 7,83 (s) |
| $H_4$ ; $H_3$ et $H_2$ | 7,47 (m) (2H) 7,81 (m) (1H) |
| $H_5$ | 7,94 (d,J = 8,5 Hz) |
| $H_6$ | 8,03 (dd,J = 8,5 - 1,5 Hz) |
| $H_8$ | 8,62 (l) |

**EXEMPLE 3 : 7-[2-(4-fluorophényl) 4-thiazolyl] α-(méthoxyméthylène) 1-naphtalène acétate de méthyle.**

A 0,32 g d'hydrure de sodium dans 10 $cm^3$ de diméthylformamide, on ajoute goutte à goutte le mélange comprenant 1 g de produit de la préparation 3, 3,3 $cm^3$ de formiate de méthyle dans 10 $cm^3$ de diméthylformamide. On maintient le mélange réactionnel sous agitation, ajoute 1,1 $cm^3$ de formiate de méthyle puis 4,2 $cm^3$ de iodométhane. On agite pendant 16 heures, verse le mélange réactionnel dans l'eau, extrait à l'éther isopropylique, évapore le solvant et chromatographie le résidu sur silice (éluant : hexane-acétate d'éthyle 7-3). On recueille 0,53 g de produit attendu. F = 181°C.

Spectre RMN ($CDCl_3$ 300 MHz)

| | |
|---|---|
| $OCH_3$ | 3,69 (s), 3,83 (s) |
| phényl | 7,17 (m), 8,06 (m) |
| $H_3$ | 7,50 (dd) |
| CH= (ΔE) | 7,85 (s) |
| H thiazole | 7,54 (s) |
| $H_8$ | 8,34 (s) |
| H aromatiques | 7,38 (dd) 1H ; 7,84 (d) 1H ; 7,92 (d) 1H ; 8,06 1H |

**Préparation du 7-[2-(4-fluorophényl) 4-thiazolyl] 1-naphtalène acétate de méthyle.**

On chauffe au reflux pendant 16 heures 1,7 g de 4-fluorophényl thioamide et 2,7 g de bromocétone dans 100 $cm^3$ de méthanol. On évapore partiellement le méthanol, reprend dans du chlorure de méthylène, lave avec une solution aqueuse de bicarbonate de sodium, décante, sèche et évapore le solvant sous pression réduite. On chromatographie le résidu sur silice (éluant : hexane-chlorure de méthylène 20-80) et obtient 2,3 g de produit attendu. F = 105°C.

**EXEMPLE 4 : 7-[2-(1,1-diméthyléthyl) 4-thiazolyl] α-(méthoxyméthylène) 1-naphtalène acétate de méthyle.**

On opère comme à l'exemple 3 en utilisant au départ 2,11 g de produit de la préparation 4 et 7,7 $cm^3$ de formiate de méthyle, puis 9,8 $cm^3$ de iodométhane. On obtient 3,1 g de produit brut que l'on chromatographie sur silice (éluant : hexane-acétate d'éthyle 8-2) et récupère 1,35 g de produit attendu. F = 113°C.

Spectre RMN ($CDCl_3$)

| | |
|---|---|
| t-Bu | 1,51 (s) |

| | |
|---|---|
| $OCH_3$ | 3,68 (s), 3,82 (s) |
| $H_5$ thiazole | 7,40 (s) |
| $H_2$ et $H_4$ | 7,35 (dd), 7,80 (d) |
| $H_3$ | 7,46 (dd) |
| $H_6$ | 8,01 (dd) |
| $H_5$ | 7,88 (d) |
| C=CH | 7,82 (s) |
| $H_8$ | 8,27 (d) |

**Préparation 4 : 7-[2-(1,1-diméthyléthyl) 4-thiazolyl] α-(méthoxy-méthylène) 1-naphtalène acétate de méthyle.**

Stade A : 2,2-diméthyl propane thioamide.

On introduit 28,5 g de pentasulfure de diphosphore dans 500 $cm^3$ de tétrahydrofuranne et ajoute par fractions 13,6 g de carbonate de sodium puis 10 g de 2,2-diméthylpropanamide. On agite 60 heures à température ambiante, verse dans l'eau, extrait au chlorure de méthylène, sèche et évapore le solvant sous pression réduite. Après chromatographie sur silice (éluant : hexane-acétate d'éthyle 6-4), on recueille 11,2 g de produit attendu. F = 117°C.

Stade B : 7-[2-(1,1-diméthyléthyl) 4-thiazolyl] α-(méthoxyméthylène) 1-naphtalène acétate de méthyle.

On chauffe au reflux pendant 16 heures 3,3 g de bromocétone obtenue comme indiqué à la préparation 1 Stade E1, 2,1 g de thioamide préparé au stade A dans 100 $cm^3$ de méthanol. On évapore partiellement le solvant, reprend dans du chlorure de méthylène, verse sur une solution d'hydrogénocarbonate de sodium, agite 5 minutes, extrait au chlorure de méthylène, sèche et évapore le solvant sous pression réduite. On chromatographie le résidu sur silice (éluant : hexane-chlorure de méthylène 3-7) et recueille 2,11 g de produit attendu. F = 61°C.

**EXEMPLE 5 : α-(méthoxyméthylène) 7-[2-[4-(trifluorométhoxy) phényl] 4-thiazolyl] 1-naphtalène acétate de méthyle.**

On opère comme à l'exemple 3 en utilisant au départ 1,28 g de produit de la préparation 5 et 3,6 $cm^3$ de formiate de méthyle puis 4,6 $cm^3$ de iodométhane. On obtient 1,78 g de produit attendu. F = 151°C après une nouvelle chromatographie sous pression (éluant : flugène-acétate d'éthyle 95-05).
Spectre RMN ($CDCl_3$ 250 MHz).

| | |
|---|---|
| Les $CH_3O$ | 3,69 ; 3,82 |
| H thiazole et | 7,87 (s) |
| CH= ΔE | 7,85 (s) |
| phényl | 7,33 - 8,08 |

**Préparation 5 : 7-[2-[4-(trifluorométhoxy) phényl] 4-thiazolyl] 1-naphtalène acétate de méthyle.**

Stade A : Chlorure de 4-(trifluorométhoxy) benzoyle.

On chauffe au reflux pendant 3 heures 18 g d'acide 4-(trifluorométhoxy) benzoïque dans 180 $cm^3$ de chlorure de thionyle. On évapore le solvant sous pression réduite et obtient 20,1 g de produit attendu.

Stade B : 4-(trifluorométhoxy) benzamide.

On ajoute lentement 20,1 g du produit obtenu au stade A dans 200 $cm^3$ d'ammoniaque en solution aqueuse (28%), essore le précipité formé et le sèche à 50°C sous pression réduite. On obtient 19,2 g de produit attendu. F = 240°C.

Stade C : 4-(trifluorométhoxy) thiobenzamide.

On opère comme au stade A de l'exemple 4 en utilisant au départ 21,7 g de pentasulfure de phosphore, 10,3 g de carbonate de sodium et en remplaçant le diméthylpropanamide par 10 g de 4-(trifluorométhoxy) benzamide préparée au stade B. On obtient 8,1 g de produit attendu. F = 134°C.

Stade D : 7-[2-[4-(trifluorométhoxy) phényl] 4-thiazolyl] 1-naphtalène acétate de méthyle.

On opère comme indiqué au stade B de l'exemple 4 en utilisant 2 g de bromocétone obtenue comme indiqué à la préparation 1 stade E1 et 2,78 g de thiobenzamide préparé au stade C ci-dessus dans 50 $cm^3$ de méthanol. Après chromatographie sur silice (éluant : hexane-acétate d'éthyle 9-1), on obtient 1,88 g de produit attendu. F = 91°C.

Spectre RMN ($CDCl_3$ 300 MHz)

| | |
|---|---|
| $OCH_3$ | 3,72 (s) |
| $=C-CH_2-C=$ | 4,17 (s) |
| $H_5$ thiazole | 7,64 (s) |
| $H_8$ | 8,63 (sl) |
| H aromatique | 7,30 à 8,15 (m) : 9H |

**EXEMPLE 6 : α-(méthoxyméthylène) 7-[2-[4-(trifluorométhyl) phényl] 4-thiazolyl] 1-naphtalène acétate de méthyle.**

On opère comme à l'exemple 3 en utilisant au départ 1,04 g de produit de la préparation 6 et 4 $cm^3$ de formiate de méthyle puis 3,9 $cm^3$ de iodométhane. On obtient 0,6 g de produit attendu. F = 167°C isomère E (rf = 2,4) et 0,08 g isomère Z (rf = 1,6).

Spectre RMN ($CDCl_3$ 250 MHz)

| | |
|---|---|
| les $CH_3O$ | 3,70 (s) et 3,82 (s) |
| $H_5$ thiazole et | 7,62 (s) |
| CH= ΔE | 7,85 (s) |
| phényl | 7,74 - 8,18 |

**Préparation 6 : 7-[2-[4-(trifluorométhyl) phényl] 4-thiazolyl] 1-naphtalène acétate de méthyle.**

Stade A : Chlorure de 4-(trifluorométhyl) benzoyle.

On opère comme indiqué à la préparation 5 stade A en utilisant au départ 24 g d'acide 4-trifluorométhyl benzoïque et obtient 26,7 g de produit attendu.

Stade B : 4-(trifluorométhyl) benzamide.

On opère comme à la préparation 5 en utilisant 26,7 g de chlorure d'acide obtenu au stade A et obtient 18,8 g de produit attendu. F = 184°C.

Stade C : 4-(trifluorométhyl) thiobenzamide.

On opère comme à la préparation 5 en utilisant 10 g de l'amide obtenue au stade B. On obtient 13,5 g de produit brut puis 5,11 g après chromatographie.

Stade D : 7-[2-[4-(trifluorométyl) phényl] 4-thiazolyl] 1-naphtalène acétate de méthyle.

On opère comme à la préparation 5 stade D à partir de 1,9 g de thioamide obtenu au stade C et 1,5 g de bromocétone obtenu comme à la préparation 1 Stade E1. On obtient 1,05 g de produit attendu. F = 112°C.

Spectre RMN ($CDCl_3$ 250 MHz)

| | |
|---|---|
| $OCH_3$ | 3,72 (s) |
| $=C-CH_2-C=$ | 4,13 (s) |
| $H_5$ thiazole | 7,74 (s) |
| $H_8$ | 8,70 (l) |

**EXEMPLE 7 : α-(méthoxyméthylène) 7-[3-(1,1-diméthyléthyl) 5-isoxazolyl ] 1-naphtalène acétate de méthyle.**

On mélange 0,63 cm³ de triéthylamine, 0,8 g de 7-éthynyl [α-(méthoxyméthylène) 1-naphtalène acétate de méthyle préparé comme indiqué ci-dessous et 10 cm³ de tétrahydrofuranne. On ajoute goutte à goutte en 1 heure à température ambiante, 0,7 g de 1-chloro 2,2-diméthyl propanal oxime préparé comme indiqué ci-dessous en solution dans 7 cm³ de tétrahydrofuranne. On agite 16 heures à température ambiante, ajoute des réactifs (triéthylamine et oxime et tétrahydrofuranne dans les mêmes proportions que ci-dessus) et agite de nouveau 24 heures. On verse la suspension dans l'eau, extrait au chlorure de méthylène, sèche et évapore le solvant. Après chromatographie du résidu sur silice (éluant : hexane-éther isopropylique 7-3 puis 5-5 puis hexane-acétate d'éthyle 8-2), on obtient 1 g de produit. On reprend le produit dans du chlorure de méthylène avec quelques gouttes d'hexane et obtient 0,8 g de produit attendu. F = 145°C.

Spectre RMN ($CDCl_3$ 250 MHz)

| | |
|---|---|
| C-tBu | 1,41 (s) |
| $OCH_3$ | 3,69 (s), 3,82 (s) |
| CH= | 7,83 (s) |

**Préparation 7A : (E) α-(méthoxyméthylène) 7-(éthynyl) 1-naphtalène acétate de méthyle**

Stade A : 7-bromo 3,4-dihydro 1-naphtalène acétate de méthyle

On chauffe à 60°C une suspension renfermant 5,8 g de poudre de zinc activé (c'est-à-dire obtenue après lavages à l'acide chlorhydrique à 20 % (en volume) puis à 5 % puis à l'eau, puis à l'éthanol et enfin à l'éther éthylique et séchage), 70 cm³ de tétrahydrofuranne anhydre, 1 cristal d'iode et 0,1 cm³ de bromoacétate de méthyle. On ajoute sous atmosphère d'azote, une solution renfermant 10 g de 7-bromotétralone (préparé selon J. Org. Chem., 27, 76 (1962)) 5 cm³ de bromoacétate de méthyle et 60 cm³ de tétrahydrofuranne anhydre. On agite ensuite le milieu réactionnel à 20°C pendant 2 heures, verse dans l'eau, neutralise par adjonction de chlorure d'ammonium et extrait à l'éther. On filtre la solution aqueuse et réextrait à l'éther. On rassemble les phases organiques, les lave avec une solution aqueuse saturée en chlorure de sodium, sèche et évapore sous pression réduite. On obtient 13,6 g d'un produit que l'on dissout dans 90 cm³ d'acide trifluoroacétique. On chauffe à 50°C pendant 30 minutes et verse dans une solution refermant 1 l d'eau et 500 cm³ d'éther. On ajoute du carbonate de potassium jusqu'à un pH voisin de 10. On décante, lave avec une solution aqueuse saturée en chlorure de sodium, sèche et évapore sous pression réduite. On obtient 11,8 g de produit que l'on chromatographie sur silice en éluant avec un mélange hexane-éther isopropylique (9-1) puis avec un mélange hexane-éther isopropylique (7-3). On obtient 9,9 g du produit recherché.

CCM (silice) éluant : hexane-éther isopropylique (8-2) rf = 0,38.

Spectre RMN (250 MHz)

| | |
|---|---|
| $CH_2$ en 3 | ≃ 2,32 (m) |
| $CH_2$ en 4 | 2,73 (t) |
| $=C-CH_2-C=O$ | 3,41 (s) |
| $CO_2CH_3$ | 3,71(s) |
| $H_2$ | 6,04 (t) |
| $H_5$ | 7,00 (d) |
| $H_8$ + $H_6$ | ≃ 7,25 (m) |

Stade B : 7-bromo 1-naphtalène acétate de méthyle

On chauffe à 90°C pendant 3 h 30 minutes une solution renfermant 9,85 g de produit préparé au stade A avec 10,3 g de dichlorodicyanobenzoquinone et 300 cm³ de toluène. On maintient le mélange réactionnel pendant 12 heures à 20°C. On filtre, et évapore sous pression réduite pour obtenir 12,7 g de produit que l'on chromatographie sur silice en éluant avec le mélange hexane-chlorure de méthylène (7-3). On obtient ainsi 5,1 g de produit recherché. F = 61°C.

Spectre de RMN (250 MHz)

| | |
|---|---|
| $COOCH_3$ | 3,66 (s) |
| $=C_6H_3-CH_2-CO$ | 3,98 (s) |
| $H_8$ | 8,1 (sl) |
| | 7,42 (m) 2H |
| H aromatiques | 7,52 (dd) 1H |

7,68 (m) 2H

Stade C : 1-(E) 7-(bromo α-méthoxyméthylène) 1-naphtalène acétate de méthyle.

On ajoute une solution renfermant 5,99 g de produit du stade B, 70 $cm^3$ de DMF et 28,5 $cm^3$ de formiate de méthyle dans une solution renfermant 2,02 g d'hydrure de sodium et 20 $cm^3$ de DMF anhydre. On maintient le mélange réactionnel sous agitation pendant 35 minutes. On verse sur une solution aqueuse d'acide chlorhydrique, extrait à l'éther, sèche, filtre et concentre. On obtient 9,54 g d'un produit que l'on reprend dans 60 $cm^3$ d'acétone. On ajoute 8,70 g de carbonate de potassium et 2,4 $cm^3$ de sulfate de diméthyle. On maintient le mélange réactionnel sous agitation à 20°C pendant 16 heures. On verse dans l'eau. On essore et rince le produit obtenu. On obtient 8,67 g d'un produit que l'on chromatographie sur silice en éluant avec le mélange hexane-chlorure de méthylène-acétone (90-5-5). On obtient ainsi 5,08 g de produit fondant à 149°C.

Stade D : (E) α-(méthoxyméthylène) 7-[2-(triméthylsilyl) éthynyl] 1-naphtalène acétate de méthyle.

On introduit 1,5 g du produit du stade C, dans un mélange anhydre de 15 $cm^3$ de triéthylamine et 10 $cm^3$ d'acétonitrile. On ajoute 0,198 g de palladium à 10 % sur charbon actif, 0,035 g d'iodure de cuivre, 0,200 g de triphénylphosphine et 0,70 $cm^3$ d'éthynyltriméthylsilane. On porte la suspension obtenue au reflux pendant 4 heures, on ajoute 0,7 $cm^3$ d'éthynyltriméthylsilane. On porte à nouveau au reflux pendant 4 heures. On refroidit, filtre et amène à sec. On obtient 2,75 g d'un produit que l'on chromatographie sur silice en éluant avec le mélange hexane-acétate d'éthyle (80-20). On isole la fraction de rf = 0,35, concentre au rotavapor. On obtient 1,35 g d'un produit que l'on recristallise dans l'isopropanol. F = 157°C.

Stade E : (E) 7-(1-hexynyl) α-(méthoxyméthylène) 1-naphtalène acétate de méthyle.

On ajoute à -50°C, 18,1 $cm^3$ d'une solution de $BU_4NF$ 1N dans le THF, dans une solution renfermant 6,15 g de (E) α-(méthoxyméthylène) 7-[2-(triméthylsilyl) éthynyl] 1-naphtalène acétate de méthyle et 180 $cm^3$ de THF. On maintient le mélange réactionnel sous agitation pendant 2 heures. On le verse sur une solution aqueuse de dihydrogéno-phosphate de potassium. On extrait au chlorure de méthylène. On sèche, évapore le solvant et obtient 6,1 g d'un produit que l'on chromatographie sur silice en éluant avec le mélange hexane-acétate d'éthyle (9-1). On obtient 4,2 g de produit recherché. F = 140°C.

**Préparation 7B : 1-chloro 2,2-diméthyl propanal oxime.**

Stade A : 2,2-diméthyl propanal oxime.

On mélange 1,2 g de chlorhydrate d'hydroxylamine dans 16 $cm^3$ de méthanol, ajoute 1,43 g d'acétate de sodium, agite 15 minutes et ajoute 1 g de 2,2-diméthyl propanal dans 5 $cm^3$ de méthanol. On chauffe 3 heures au reflux, agite 16 heures à température ambiante, verse la suspension dans l'eau, extrait au chlorure de méthylène, sèche et évapore le solvant. On obtient 1,3 g de produit attendu.

Stade B : 1-chloro 2,2-diméthyl propanal oxime.

On chauffe à 25°/30°C 1,3 g de produit obtenu au stade A dans 13 $cm^3$ de diméthylformamide, ajoute 0,19 g de N-chlorosuccinimide, agite 20 minutes à 30°C, ajoute de nouveau 1,5 g de N-chlorosuccinimide et maintient sous agitation 5 heures à 45°C. On verse dans l'eau, extrait à l'éther isopropylique, sèche et évapore le solvant. On obtient 1,8 g de produit attendu.

**EXEMPLE 8 : α-(méthoxyméthylène) 7-[3-(trifluorométhyl) 5-isoxazolyl] 1-naphtalène acétate de méthyle.**

On mélange 4 $cm^3$ d'éther, 0,37 $cm^3$ de triéthylamine, 0,6 g de 7-éthynyl [α-(méthoxyméthylène) 1-naphtalène acétate de méthyle préparé comme indiqué ci-dessus à la préparation 7 et 6 $cm^3$ de tétrahydrofuranne. On ajoute goutte à goutte en 2 heures à température ambiante 0,84 g de bromure de trifluoroacétaldéhyde oxime préparé comme indiqué dans Bull. Chem. Soc. Jpn. 57, 2184-2187 (1984) en solution dans 8 $cm^3$ de tétrahydrofuranne. On agite 16 heures à température ambiante, verse la suspension dans l'eau, extrait au chlorure de méthylène, sèche et élimine le solvant sous pression réduite. On obtient 1 g de produit brut que l'on chromatographie sur silice (éluant : hexane-acétate d'éthyle 8-2 puis hexanechlorure de méthylène 5-5). On

obtient après recristallisation dans l'isopropanol 0,17 g de produit attendu. F = 158°C.

Spectre RMN ($CDCl_3$ 300 MHz)

| | |
|---|---|
| $OCH_3$ | 3,71 (s), 3,83 (s) |
| H isoxazole | 6,82 (s) |
| $H_3$ | 7,40 (t) |
| H aromatiques | 7,43 (dd), 7,79 (dd), 7,87 (dd), 7,98 (dd) |
| $H_8$ | 8,24 (s) |
| CH=C ΔE | 7,86 (s) |

En opérant comme indiqué aux exemples ci-dessus, on a préparé les produits suivants :

**EXEMPLE 9 : (E) α-(méthoxyméthylène) 7-[2-(méthylsulfinyl) 4-thiazolyl] 1-naphtalène acétate de méthyle.**

**EXEMPLE 10 : (E) α-(méthoxyméthylène) 7-[5-(nitro 2-thiazolyl)] 1-naphtalène acétate de méthyle.**

F = 180°C.

**EXEMPLE 11 : (E) 7-[3-(phényl 5-isoxazolyl) α-(méthoxyméthylène) 1-naphtalène acétate de méthyle.**

F = 175°C.

**EXEMPLE 12 : (E) 7-[2-[1,3-benzodioxol-5-yl] 4-thiazolyl] α-(méthoxyméthylène) 1-naphtalène acétate de méthyle.**

F = 159°C.

**EXEMPLE 13 : (E) 7-[2-(1-méthyléthyl) 4-thiazolyl] α-(méthoxyméthylène) 1-naphtalène acétate de méthyle.**

F = 123°C.

**EXEMPLE 14 : (E) 7-[3-(1-méthyléthyl) 5-isoxazolyl] α-(méthoxyméthylène) 1-naphtalène acétate de méthyle.**

F = 135°C.

**EXEMPLE 15 : (E) 7-[3-(3,4-dichlorophényl) 5-isoxazolyl] α-(méthoxyméthylène) 1-naphtalène acétate de méthyle.**

F = 222°C.

**EXEMPLE 16 : (E) 7-[3-pentyl 5-isoxazolyl] α-(méthoxyméthylène) 1-naphtalène acétate de méthyle.**

F = 120°C.

**EXEMPLE 17 : (E) 7-[3-(4-fluorophényl 5-isoxazolyl] α-(méthoxyméthylène) 1-naphtalène acétate de méthyle.**

F = 194°C.

**EXEMPLE 18 : (E) 7-[3-(1-méthylbutyl 5-isoxazolyl] α-(méthoxyméthylène) 1-naphtalène acétate de méthyle.**

F = 111°C.

**EXEMPLE 19 : (E) 7-[2'-(1,1-diméthyléthyl (2,4'bithiazol)-4-yl] α-(méthoxyméthylène) 1-naphtalène acétate de méthyle.**

F = 173°C.

**EXEMPLE 20 : (E) 7-[2'-(1-méthyléthyl (2,4'bithiazol)-4-yl] α-(méthoxyméthylène) 1-naphtalène acétate de méthyle.**

F = 144°C.

**EXEMPLE 21 : (E) 7-[3-[4-(trifluorométhoxy) phényl] 5-isoxazolyl] α-(méthoxyméthylène) 1-naphtalène acétate de méthyle.**

F = 183°C.

**EXEMPLE 22 : (E) 7-[5-(trifluorométhyl) 1,2,4-oxadiazol-3-yl] α-(méthoxyméthylène) 1-naphtalène acétate de méthyle.**

On agite à 80°C pendant 1 heure 2,8 g de produit obtenu à la préparation ci-dessous dans 30 $cm^3$ de terbutoxy bis(diméthylamino) méthyle. On verse le mélange dans 30 $cm^3$ d'acide chlorhydrique concentré glacé. On agite 3 heures à 20-25°C, extrait au chlorure de méthylène, lave à l'eau jusqu'à neutralité, sèche et évapore le solvant. On obtient 3,5 g de produit brut auquel on ajoute 40 $cm^3$ d'acétone, 13 g de carbonate de potassium et 6 $cm^3$ d'iodure de méthyle. On agite 2 heures à température ambiante, filtre, lave à l'acétone et élimine le solvant. Après chromatographie sur silice (éluant : chlorure de méthylène-hexane 8-2), on obtient 2,2 g de produit attendu. F = 127°C.

**Préparation 22 : 7-[5-(trifluorométhyl) 1,2,4-oxadiazol-3-yl] 1-naphtalène acétate de méthyle.**

Stade A : 7-nitro 3,4-dihydronaphtalène 1-acétate de méthyle.

On mélange 60 g de zinc dans 100 $cm^3$ de tétrahydrofuranne, amorce la réaction à l'aide d'iode, agite sous reflux et ajoute en 1 heure et demie 78 $cm^3$ de bromoacétate de méthyle, maintient 15 minutes de reflux, agite 1 heure et laisse revenir à température ambiante. On ajoute alors à température ambiante 35 g de 7-nitrotétralone dans 200 $cm^3$ de tétrahydrofuranne, agite 1 heure, coule dans un mélange d'eau et d'acide chlorhydrique (pH = 1), agite 1 heure et extrait au chlorure de méthylène. On sèche, évapore le solvant et obtient 57,5 g de produit brut que l'on chauffe à 55-60°C avec 100 $cm^3$ d'acide trifluoroacétique. On verse dans de l'eau glacée, extrait au chlorure de méthylène, lave avec une solution aqueuse de bicarbonate de sodium, sèche et évapore les solvants. Après chromatographie sur silice (éluant : chlorure de méthylène), on obtient 23,8 g de produit attendu.

Stade B : 7-nitro naphtalène 1-acétate de méthyle.

On refroidit à 0°C 28,5 g de produit obtenu comme au stade A dans 150 $cm^3$ de chlorure de méthylène, ajoute en 30 minutes 18,5 g de brome en solution dans 50 $cm^3$ de chlorure de méthylène, agite 15 minutes à 0°C, introduit goutte à goutte 53 g de 1,8-diazabicyclo[5,4,0]undec-7-ène, puis agite 1 heure à température ambiante. On verse dans l'eau glacée, ajoute de l'acide chlorhydrique jusqu'à pH 1, décante, extrait au chlorure de méthylène, lave à l'eau, sèche, évapore le solvant et récupère 17 g de produit attendu. F = 92,2°C.

Stade C : 7-amino naphtalène 1-acétate de méthyle.

On hydrogène à 20°C, 15 g de produit obtenu au stade B dans 15 $cm^3$ de méthanol en présence de 100 mg de palladium sur charbon à 10%. On filtre, rince au méthanol, évapore le solvant, chromatographie le résidu sur silice (éluant : chlorure de méthylène-acétate d'éthyle 95-5) et obtient 12,7 g de produit attendu.

Stade D : 7-nitrile naphtalène 1-acétate de méthyle.

On agite pendant 30 minutes 11 g du produit obtenu au stade C dans 150 $cm^3$ d'eau en présence de 12 $cm^3$ d'acide chlorhydrique. On ajoute en 30 minutes 4,2 g de nitrite de sodium en solution dans 50 $cm^3$ d'eau,

agite 30 minutes et obtient la suspension de sel de diazonium. On refroidit à 0°C 15,3 g de sulfate cuivrique dans 60 $cm^3$ d'eau, ajoute en 15 minutes 18 g de cyanure de potassium dans 100 $cm^3$ d'eau, agite, ajoute à 0°C 50 $cm^3$ d'ammoniaque, agite 15 minutes et ajoute la suspension de sel de diazonium obtenue ci-dessus. Après 1 heure d'agitation à température ambiante, on extrait au chlorure de méthylène, sèche, évapore le solvant et chromatographie le résidu sur silice (éluant : chlorure de méthylène). On obtient 7 g de produit attendu. F = 85°C.

Stade E : 7-[amino (hydroxyimino) méthyle] acétate de méthyle

On chauffe au reflux pendant 4 heures, 3 g de produit obtenu au stade D, 1,4 g de chlorhydrate d'hydroxylamine, 2,7 g d'acétate de sodium dans 65 $cm^3$ d'éthanol 95. On évapore le solvant, reprend le résidu au chlorure de méthy-lène, sèche et élimine le solvant sous pression réduite. On obtient 4,07 g de produit utilisé tel quel pour le stade suivant.

Stade F : 7-[5-(trifluorométhyl) 1,2,4-oxadiazol-3-yl] 1-naphtalène acétate de méthyle.

On chauffe à 80°C pendant 2 heures, 4 g de produit obtenu au stade E dans 60 $cm^3$ de toluène et 6,5 $cm^3$ d'anydride trifluoroacétique. On élimine le solvant, chromatographie le résidu sur silice (éluant : chlorure de méthylène) et obtient 2,8 g de produit attendu.

**EXEMPLE 23 : (E) 7-[2'-éthyl) 2,4'-bithiazol-4-yl] α-(méthoxyméthylène) 1-naphtalène acétate de méthyle.**

F = 193°C.

**EXEMPLE 24 : (E) 7-[2-méthylthio) 4-thiazolyl] α-(méthoxyméthylène) 1-naphtalène acétate de méthyle.**

F = 185°C.

**EXEMPLE 25 : (E) 7-[3-(4-méthoxyphényl) 5-isoxazolyl] α-(méthoxyméthylène) 1-naphtalène acétate de méthyle.**

F = 198°C.

**EXEMPLE 26 : (E) 7-[2-(diméthylamino) 4-thiazolyl] α-(méthoxyméthylène) 1-naphtalène acétate de méthyle.**

F = 169°C.

**EXEMPLE 27 : (E) 7-[4-(fluorophényl) 5-méthyl] 2-thiazolyl] α-(méthoxyméthylène) 1-naphtalène acétate de méthyle.**

Stade A : 7-(tributylétain) α-(méthoxyméthylène) 1-naphtalène acétate de méthyle.

On ajoute progressivement 41 $cm^3$ d'hexabutyldistannane dans un mélange comprenant 4,37 g de 1-(E)-7-(bromo α-méthoxyméthylène 1-naphtalène acétate de méthyle préparé comme indiqué à la préparation 7 dans 60 $cm^3$ de toluène et 0,6 g de tétrakis(triphénylphosphine) palladium. On chauffe le milieu réactionnel pendant 6 heures à 80°C, évapore le solvant, chromatographie le résidu sur silice (éluant : hexane-chlorure de méthylène 6-4), concentre les fractions de rf = 0,19 et recueille 4,59 g de produit attendu.

Stade B : (E) 7-[4-(fluorophényl) 5-méthyl] 2-thiazolyl] α-(méthoxyméthylène) 1-naphtalène acétate de méthyle.

On chauffe 20 heures au reflux 1,53 g de 2-bromo 4-(4-fluorophényl) 5-méthyl thiazole, 2 g du dérivé stannylé préparé au stade A, 0,132 g de chlorure de bis(triphénylphosphine) palladium dans 50 $cm^3$ de dioxanne. On filtre, lave le filtrat avec un mélange comprenant 50 $cm^3$ d'acétate d'éthyle et 50 $cm^3$ d'une solution aqueuse de fluorure de potassium (1M). On filtre le fluorure de tributyl étain précipité, lave de nouveau le filtrat, évapore

le solvant, chromatographie le résidu sur silice (éluant : hexane-chlorure de méthylène 25-75) et obtient 0,38 g de produit attendu.
F = 160°C.

**Préparation 27 : 2-bromo 4-(4-fluorophényl) 5-méthyl thiazole.**

Stade A : α-bromo 4-fluoro propiophénone.

On refroidit à -20°C 9,1 $cm^3$ de parafluoropropiophénone, 100 $cm^3$ de chlorure de méthylène, 200 $cm^3$ de pentane et 13,7 $cm^3$ d'hexaméthyldisilazane puis ajoute goutte à goutte 9,9 $cm^3$ de iodotriméthylsilane. On laisse revenir à température ambiante, agite 20 heures, filtre, évapore le solvant sous pression réduite, dissout l'éther d'énol silylé obtenu dans 200 $cm^3$ de tétrahydrofuranne et ajoute 12,86 g de N-bromosuccinimide. On évapore le solvant, reprend au chlorure de méthylène, lave à l'aide d'une solution aqueuse molaire de thiosulfate de sodium, décante, sèche la phase organique et évapore le solvant. Le produit brut est utilisé tel quel pour le stade suivant.

Stade B : 2-amino 4-(4-fluorophényl) 5-méthyl thiazole.

On chauffe au reflux pendant 3 heures 24,3 g du dérivé bromé du stade A et 5 g de thiourée dans 200 $cm^3$ d'éthanol puis ajoute après 16 heures d'agitation à 20°C, 1 g de thiourée. On chauffe de nouveau 2 heures au reflux, évapore l'éthanol, reprend le résidu au chlorure de méthylène, lave avec une solution aqueuse saturée de bicarbonate de sodium, décante, sèche la phase organique et évapore le solvant. On obtient 13,15 g de produit brut que l'on chromatographie sur silice (éluant : hexane-acétate d'éthyle 8-2). F = 155°C.

Stade C : 2-bromo 4-(4-fluorophényl) 5-méthyl thiazole.

On mélange 5 g de produit obtenu au stade B, 3,75 g de bromure de cuivre II dans 75 $cm^3$ d'acétonitrile. On ajoute en 30 minutes 2,85 $cm^3$ de nitrite de terbutyle dans 25 $cm^3$ d'acétonitrile, agite 1 heure et demie, verse dans un mélange eau-chlorure de méthylène, acidifie à pH = 1 à l'aide d'acide chlorhydrique, extrait au chlorure de méthylène, sèche et évapore le solvant. On obtient 6,9 g de produit brut attendu que l'on chromatographie sur silice (éluant : chlorure de méthylène). On récupère 4,7 g de produit pur.

**EXEMPLE 28 : (E) 7-[2-[(1-méthyléthyl) thio] 4-thiazolyl] α-(méthoxyméthylène) 1-naphtalène acétate de méthyle.**

F = 100°C.

**EXEMPLE 29 : (E) 7-[5-(trifluorométhyl) 1,3,4-thiadiazol-2-yl] α-(méthoxyméthylène) 1-naphtalène acétate de méthyle.**

On opère comme au stade B de l'exemple 27 à partir de 6 g du dérivé stannylé préparé au stade A et 2 g de 2-bromo 5-trifluorométhyl 1,3,4-thiazole préparé comme indiqué dans le brevet DE. 2533604 (1977). On obtient 5,87 g de produit brut que l'on chromatographie sur silice (éluant : hexane-tétrahydrofuranne 9-1) et recristallise dans l'isopropanol. On obtient 3,03 g de produit attendu. F = 177°C.

**EXEMPLE 30 : (E) 7-[2-(méthylsulfonyl) 4-thiazolyl] α-(méthoxyméthylène) 1-naphtalène acétate de méthyle.**

F = 215°C.

**EXEMPLE 31 : (E) 7-[5-[4-(trifluorométhoxy) phényl] 1,2,4-oxadiazol-3-yl] α-(méthoxyméthylène) 1-naphtalène acétate de méthyle.**

F = 156°C.

**EXEMPLE 32 : (E) 7-[5-[4-(fluorophényl) 1,2,4-oxadiazol-3-yl] α-(méthoxyméthylène) 1-naphtalène acétate de méthyle.**

F = 160°C.

**EXEMPLE 33 : Préparation d'un concentré émulsifiable.**

On a préparé un concentré émulsifiable renfermant comme principe actif, le produit de l'exemple 1.

**ETUDE D'ACTIVITE DES PRODUITS DE L'INVENTION**

I) Activité insecticide :

a) Etude de l'effet sur larves de Spodoptera littoralis par contact et ingestion.

On utilise des larves du stade L3 de Spodoptera littoralis. On opère à 22°C dans des conditions d'humidité relative de 50 %. On utilise des boites de PETRI renfermant un rond de papier filtre humidifié, dans chaque boîte on place deux feuilles de haricot traitées par une solution hydroacétonique (50-50) renfermant le produit à tester.

On fait le comptage des larves mortes au bout de 7 jours. On exprime le résultat en $DL_{50}$ en ppm.

Dès la dose de 100 ppm, les produits des exemples 1, 3, 4, 5, 6, 8, 12, 19, 20 et 22 conduisent à une efficacité ≧ 90 %.

b) Etude de l'activité sur Aphis craccivora :

Des plants de fèves sont traités par trempage des feuilles dans une solution hydroacétonique de matière active (50 % acétone, 50 % eau) puis séchées sous hotte aspirante.

Les feuilles sont ensuite infestées : 20 femelles d'Aphis craccivora adultes par feuille et maintenues à 22°C sous plafond lumineux.

Les contrôles de mortalité sont effectués au bout de 48 heures.

Dès la dose de 100 ppm, les produits des exemples 3, 5, 8, 10, 11, 14, 20 et 22 conduisent à une efficacité ≧ 90 %.

c) Etude de l'effet létal sur Musca domestica

On utilise des mouches femelles âgées de 6 jours pesant de 18 à 20 mg.

On opère à 22°C dans des conditions d'humidité relative de 50 %.

Les produits sont administrés par application topique de solution acétonique sur le thorax dorsal des insectes. Les $DL_{50}$ sont exprimés en ppm.

Les résultats obtenus sont les suivants :

. Produit de l'exemple 1 .......... 25 ppm $\leq DL_{50} <$ 250 ppm

. Produit de l'exemple 2 ......... 250 ppm $\leq DL_{50} <$ 1000 ppm

II) Activité acaricide sur Tetranychus urticae.

On utilise des plants de haricot comportant 2 feuilles infestées de 30 femelles de Tetranychus Urticae par feuille et mis sous bonette aérée sous plafond lumineux en lumière constante. Les plants sont traités au pistolet Fisher : 4 ml de solution toxique par plant d'un mélange à volume égal d'eau et d'acétone. On laisse sécher pendant une 1/2 heure puis on procède à l'infestation. Les contrôles de mortalité sont effectués au bout de 3 jours.

On recherche la dose $DL_{50}$. Les résultats sont exprimés en ppm.

Dès la dose de 100 ppm, les produits des exemples 2, 3, 4, 5, 6, 7, 8, 12, 13, 14, 18, 19, 20 et 22 conduisent à une efficacité ≧ 97 %.

**Revendications**

**1.-** Les composés de formule générale (I) :

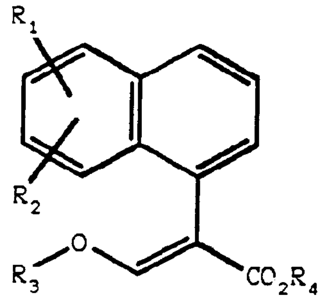

(I)

dans laquelle :

- $R_1$ représente un atome d'hydrogène, un radical hydroxyle, un atome d'halogène, un radical $NO_2$, $NH_2$, C≡N, $CF_3$, $OCF_3$ ou $SCF_3$, un radical $OR'_1$, $SR'_2$ ou $N(R'_3)_2$, $R'_1$, $R'_2$ et $R'_3$ représentant un radical alkyle, alkényle ou alkynyle renfermant jusqu'à 8 atomes de carbone ou un radical aryle renfermant jusqu'à 14 atomes de carbone, éventuellement substitué par un ou plusieurs substituants choisis parmi les atomes d'halogène et les radicaux $NO_2$, $NH_2$, C≡N, $CF_3$, $OCF_3$ et $SCF_3$,
- $R_2$ représente un radical hétérocyclique à 5 chaînons renfermant au moins 2 hétéroatomes et relié au noyau naphtyl par une liaison carbone-carbone, éventuellement substitué par un ou plusieurs atomes groupes Z, Z représentant un atome d'halogène, un radical alkyle, alkényle ou alkynyle renfermant jusqu'à 8 atomes de carbone linéaire ou ramifié, un radical hydroxyle, $NO_2$, $NH_2$, C≡N, $CF_3$, $OCF_3$ ou $SCF_3$, $OR''_1$, $SR''_2$, $SOR''_2$, $SO_2R''_2$, $NR''_3$, $R''_1$, $R''_2$ et $R''_3$ représentant un radical alkyle, alkényle ou alkynyle renfermant jusqu'à 8 atomes de carbone ou Z représente un radical hétérocyclique à 5 ou 6 chaînons ou un radical aryle renfermant jusqu'à 14 atomes de carbone éventuellement substitué par un ou plusieurs substituants choisis parmi les atomes d'halogène et les radicaux $NO_2$, $NH_2$, C≡N, $CF_3$, $OCF_3$, $SCF_3$, un radical méthylènedioxy ou un radical $OR''_1$, $SR''_2$, $SOR''_2$, $SO_2R''_2$ ou $NR''_3$, $R''_1$, $R''_2$ et $R''_3$ conservant leur signification précédente,
- $R_3$ et $R_4$, identiques ou différents, représentent un radical alkyle renfermant jusqu'à 8 atomes de carbone linéaire, ramifié ou cyclique éventuellement substitué par un ou plusieurs atomes d'halogène.

**2.**- Les composés de formule (I) tels que définis à la revendication 1, dans lesquels $R_3$ représente un radical méthyle.

**3.**- Les composés de formule (I) tels que définis à la revendication 1 ou 2 dans lesquels $R_4$ représente un radical méthyle.

**4.**- Les composés de formule (I) tels que définis à l'une quelconque des revendications 1 à 3 dans lesquels $R_1$ représente un atome d'hydrogène.

**5.**- Les composés de formule (I) tels que définis à l'une quelconque des revendications 1 à 4 dans lesquels $R_2$ représente un radical thiazolyle, isoxazolyle ou oxadiazyle éventuellement substitué par un ou plusieurs des substituants indiqués à la revendication 1.

**6.**- Les composés de formule (I) tels que définis à la revendication 5 dans lesquels $R_2$ représente un radical thiazolyle substitué par un radical phényle substitué par un ou plusieurs atomes d'halogène ou par un ou plusieurs radicaux alkyle ou alkoxy éventuellement substitués par un ou plusieurs atomes d'halogène.

**7.**- Les composés de formule (I) tels que définis à la revendication 6 caractérisés en ce que $R_2$ représente un radical thiazolyle substitué par un radical phényle substitué par un ou plusieurs atomes de fluor ou de chlore, par un ou plusieurs radicaux $CF_3$ ou $OCF_3$.

**8.**- Les composés de formule (I) tels que définis à la revendication 5 dans lesquels $R_2$ représente un radical thiazolyle substitué par un ou plusieurs radicaux alkyles éventuellement substitués par un ou plusieurs atomes d'halogène.

**9.**- Les composés de formule (I) tels que définis à la revendication 5, dans lesquels $R_2$ représente un radical thiazolyle substitué par un radical $CF_3$.

**10.**- Les composés de formule (I) tels que définis à la revendication 5 dans lesquels $R_2$ représente un radical bithiazolyle éventuellement substitué par un ou plusieurs radicaux alkyle.

**11.**- Les composés de formule (I) tels que définis à la revendication 5 dans lesquels $R_2$ représente un radical isoxazolyle substitué par un ou plusieurs radicaux alkyles éventuellement substitués par un ou plusieurs atomes d'halogène.

**12.**- Les composés de formule (I) tels que définis à la revendication 5 dans lesquels $R_2$ représente un radical oxadiazyle substitué par un ou plusieurs radicaux alkyles éventuellement substitués par un ou plusieurs atomes d'halogène.

**13.**- Les composés de formule (I) dont les noms suivent :
- (E) 7-[2-(3,4-dichlorophényl) 4-thiazolyl] α-(méthoxyméthylène) 1-naphtalène acétate de méthyle,
- (E) α-(méthoxyméthylène) 7-[2-(trifluorométhyl) 4-thiazolyl] 1-naphtalène acétate de méthyle,
- (E) 7-[2-(4-fluorophényl) 4-thiazolyl] α-(méthoxyméthylène) 1-naphtalène acétate de méthyle,
- (E) 7-[2-[4-(trifluorométhoxy) phényl] 4-thiazolyl] α-(méthoxyméthylène) 1-naphtalène acétate de méthyle,
- (E) α-(méthoxyméthylène) 7-[3-(trifluorométhyl) 5-isoxazolyl) 1-naphtalène acétate de méthyle,
- (E) α-(méthoxyméthylène) 7-[2'-(1-méthyléthyl) 2,4'-bithiazole) 4-yl] 1-naphtalène acétate de méthyle,
- (E) α-(méthoxyméthylène) 7-[5-(trifluorométhyl) 1,2,4-oxadiazol -3-yl] 1-naphtalène acétate de méthyle.

**14.**- Les compositions destinées à la lutte contre les parasites des végétaux, les parasites des locaux et les parasites des animaux à sang chaud, caractérisées en ce qu'elles renferment comme principe actif au moins un des produits définis à l'une quelconque des revendications 1 à 12.

**15.**- Les compositions destinées à la lutte contre les parasites des végétaux, les parasites des locaux et les parasites des animaux à sang chaud, caractérisées en ce qu'elles renfermant comme principe actif au moins un des produits définis à la revendication 13.

**16.**- Les compositions insecticides renfermant comme principe actif au moins l'un des produits définis à l'une quelconque des revendications 1 à 13.

**17.**- Les compositions acaricides renfermant comme principe actif au moins l'un des produits définis à l'une quelconque des revendications 1 à 13.

**18.**- Les compositions nématicides renfermant comme principe actif au moins l'un des produits définis à l'une quelconque des revendications 1 à 13.

**19.**- Les compositions acaricides destinées à la lutte contre les parasites des animaux à sang chaud, notamment contre les tiques et les gales, caractérisées en ce qu'elles renferment comme principe actif, au moins l'un des produits définis à l'une quelconque des revendications 1 à 13.

**20.**- Associations douées d'activité insecticide, acaricide ou nématicide, caractérisées en ce qu'elles contiennent comme matière active, d'une part un au moins des composés de formule générale (I) telle que définie à la revendication 1, et d'autre part, un au moins des esters pyréthrinoïdes choisis dans le groupe constitué par les esters d'alléthrolone, d'alcool 3,4,5,6-tétrahydrophtalimido méthylique, d'alcool 5-benzyl 3-furyl méthylique, d'alcool 3-phénoxy benzylique et d'alcool alpha-cyano 3-phénoxy benzyliques des acides chrysanthémiques, par les esters d'alcool 5-benzyl 3-furyl méthylique des acides 2,2-diméthyl 3-(2-oxo 3-tétrahydrothiophénylidène méthyl) cyclopropanecarboxyliques, par les esters d'alcool 3-phénoxy benzylique et d'alcool alpha-cyano 3-phénoxy benzyliques des acides 2,2-diméthyl 3-(2,2-dichlorovinyl) cyclopropanecarboxyliques, par les esters d'alcools alpha-cyano 3-phénoxy benzyliques d'acides 2,2-diméthyl 3-(2,2-dibromovinyl) cyclopropanecarboxyliques, par les esters d'alcool 3-phénoxy benzylique des acides 2-parachlorophényl 2-isopropyl acétiques par les esters d'alléthrolone, d'alcool 3,4,5,6-tétrahydrophtalimido méthylique, d'alcool 5-benzyl 3-furyl méthylique, d'alcool 3-phénoxy benzylique et d'alcools alpha-cyano 3-phénoxybenzyliques des acides 2,2-diméthyl 3-(1,2-2,2-tétrahaloéthyl) cyclopropanecarboxyliques, dans lesquels "halo" représente un atome de fluor, de chlore ou de brome, étant entendu que les copules acides et alcools des esters pyréthrinoïdes ci-dessus peuvent exister sous toutes leurs formes stéréoisomères possibles.

**21.**- Les compositions telles que définies à l'une des revendications 14 à 20, caractérisées en ce qu'elles renferment en outre un synergiste des pyréthrinoïdes.

**22.**- Procédé de préparation des composés de formule (I) tels que définis à l'une quelconque des revendications 1 à 12, caractérisé en ce que l'on soumet un composé de formule (II) :

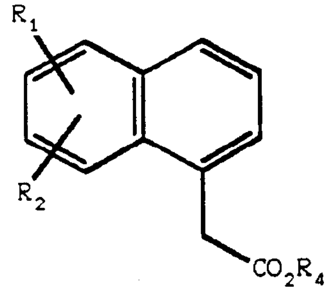

(II)

dans laquelle $R_1$, $R_2$ et $R_4$ conservent la même signification que précédemment, à l'action d'un agent de formylation puis à l'action d'un agent d'alkylation susceptible d'introduire le radical $R_3$, $R_3$ étant défini comme précédemment pour obtenir le composé de formule (I) correspondant.

**23.-** Procédé de préparation des composés de formule (I) tels que définis à l'une quelconque des revendications 1 à 12, caractérisé en ce que l'on soumet un produit de formule (II') :

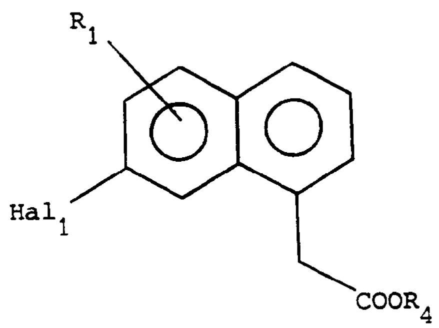

(II')

dans laquelle $R_1$ a la signification indiquée précédemment à l'exception d'un atome d'halogène, $R_4$ a la signification déjà indiquée et $Hal_1$ représente un atome d'halogène à l'action d'un agent de formylation puis à l'action d'un agent d'alkylation susceptible d'introduire le radical $R_3$, $R_3$ étant défini comme précédemment pour obtenir le composé de formule (III) :

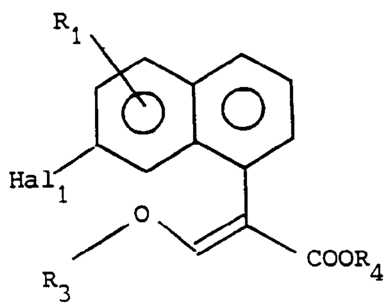

(III)

dans laquelle $R_1$, $R_3$, $R_4$ et $Hal_1$ ont la signification déjà indiquée, que l'on soumet à l'action d'un réactif stannylé en présence d'un catalyseur au palladium et d'un dérivé de triphénylphosphine pour obtenir un produit de formule (IV) :

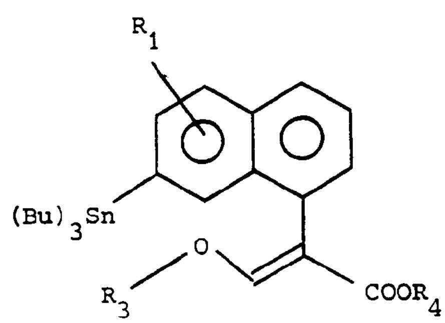

(IV)

dans laquelle $R_1$, $R_3$ et $R_4$ ont la signification déjà indiquée que l'on soumet en présence d'un catalyseur au palladium et d'un dérivé de triphénylphosphine à l'action d'un produit de formule (V) :

$R_2$-X (V)

dans laquelle X représente un atome d'halogène et $R_2$ a la signification indiquée précédemment, pour obtenir le composé de formule (I) correspondant.

**24.-** Procédé de préparation des composés de formule (I) tels que définis à la revendication 1 dans laquelle

$R_1$, $R_3$ et $R_4$ ont la signification indiqué&e ci-dessus et $R_2$ représente un radical isoxazolyle éventuellement substitué par un groupe Z dans lequel Z a la signification indiquée précédemment, caractérisé en ce que l'on soumet un produit de formule (VI) :

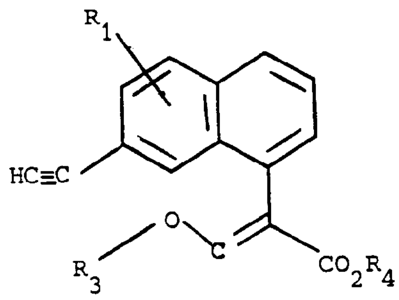

(VI)

en milieu basique, à l'action d'une oxime de formule

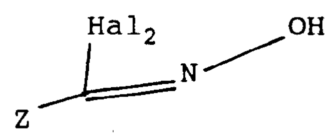

dans laquelle $Hal_2$ représente un atome d'halogène et Z a la signification indiquée à la revendication 1 pour obtenir le composé de formule (I) correspondant.

**25.-** A titre de produits chimiques nouveaux, les composés de formules (II), (II'), (III) et (IV) tels que définis aux revendications 22 et 23.

Office européen des brevets

# RAPPORT DE RECHERCHE EUROPEENNE

Numero de la demande

EP 92 40 2720

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | EP-A-0 402 246 (ROUSSEL-UCLAF)<br>* revendications 1-10,20,21 *<br>--- | 1,14-19 | C07D277/30<br>A01N43/78<br>C07D261/08 |
| Y | EP-A-0 267 734 (IMPERIAL CHEMICAL INDUSTRIES PLC)<br>* revendications 1-5,8-10 * | 1,14-19 | C07D271/06<br>C07D285/12<br>C07D277/36 |
| A | * revendications 6,7 *<br>--- | 23 | C07D277/42<br>A01N43/80 |
| A | EP-A-0 178 826 (IMPERIAL CHEMICAL INDUSTRIES PLC)<br>*Page 13,composé 60*<br>* revendications 1-5,18-21 *<br>----- | 1,14-19 | C07C69/734<br>C07C69/65<br>C07F7/22<br>C07C69/732<br>A01N43/82 |

| DOMAINES TECHNIQUES RECHERCHES (Int. Cl.5) |
|---|
| C07D<br>C07C<br>C07F |

Le présent rapport a été établi pour toutes les revendications

| Lieu de la recherche | Date d'achèvement de la recherche | Examinateur |
|---|---|---|
| LA HAYE | 06 JANVIER 1993 | HENRY J.C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)